# EUROPÄISCHE PATENTANMELDUNG

(11) **EP 0 657 417 A1**
(43) Veröffentlichungstag der Anmeldung: **14.06.1995**
(21) Anmeldenummer: 94118289.1
(22) Anmeldetag: 21.11.1994
(51) Int. Cl.: C07C 231/12, C07C 233/15, C25B 3/04

(54) **Verfahren zur Herstellung von 3-Alkyl-2,6-dichloracylaniliden durch elektrolytische Debromierung von 3-Alkyl-4-brom-2,6-dichloracylaniliden**

(30) Priorität: 01.12.1993 DE 4340896
(71) Anmelder: HOECHST AKTIENGESELLSCHAFT, D-65929 Frankfurt am Main (DE)
(72) Erfinder: Karcher, Thomas, Dr., D-65719 Hofheim (DE)

(57) **Zusammenfassung**

Verfahren zur Herstellung von 3-Alkyl-2,6-dichloracylaniliden der allgemeinen Formel I 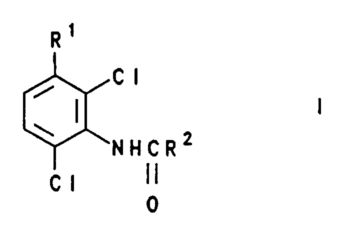
worin R¹, R² unabhängig voneinander (C₁-C₄)-Alkyl bedeuten, indem man Bromalkylacylanilide der allgemeinen Formel II 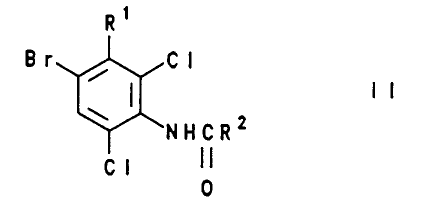
worin R¹, R² die oben angegebene Bedeutung besitzen, elektrolytisch debromiert.

## Beschreibung

Die Erfindung beschreibt ein Verfahren zur Herstellung von 3-Alkyl-2,6-dichloracylaniliden durch elektrolytische Debromierung von 3-Alkyl-4-brom-2,6-dichloracylaniliden.

Alkylacylanilide sind wertvolle Zwischenprodukte für die Herstellung von physiologisch wirksamen Verbindungen (US 5 006 656, US 4 937 350).

3-Alkyl-2,6-dichlor-acylanilide sind nicht durch direkte Chlorierung von 3-Alkylacylaniliden zugänglich, da hierbei immer auch das entsprechende 4-Chlor-substituierte Produkte gebildet wird. Hingegen können 3-Alkyl-4-brom-2,6-dichloracylanilide in hoher Ausbeute durch Bromierung und anschließende Chlorierung erhalten werden.

Reduktive Dehalogierungen am Aromaten sind in der Literatur beschrieben. Hierzu werden toxische Reagentien wie Triphenylzinnhydrid (J. Org. Chem. 1963, 28, 2332) oder teure Reagentien wie z.B. Lithiumaluminiumhydrid (J. Chem. Res. 1990, 190) benötigt, was diese Verfahren für technische Zwecke ungeeignet macht. Die katalytische Dehalogenierung mit Wasserstoff verläuft wenig selektiv.

Es bestand somit ein großer Bedarf nach einem Verfahren, das 3-Alkyl-2,6-dichloracylanilide in hoher Ausbeute und Reinheit zugänglich macht.

Diese Aufgabe wird gelöst durch ein Verfahren zur Herstellung von 3-Alkyl-2,6-dichloracylaniliden der allgemeinen Formel I 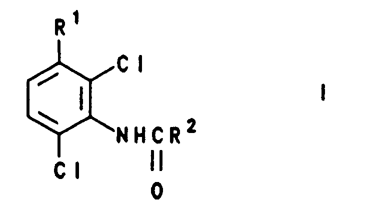
worin R¹, R² unabhängig voneinander (C₁-C₄)-Alkyl bedeuten, dadurch gekennzeichnet, daß man Bromalkylacylanilide der allgemeinen Formel II 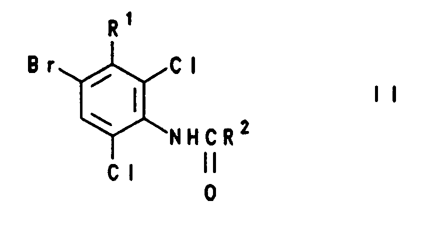
worin R¹, R², die oben angegebene Bedeutung besitzen, elektrolytisch debromiert.

Von besonderer Bedeutung ist das Verfahren für die Verbindungen der Formel I, worin R¹ und R² gleich Methyl bedeuten.

Als Reaktionsapparatur für die Elektrolyse dient eine Vorrichtung, die durch ein Diaphragma oder eine Membran in einen Anoden- und einen Kathodenhalbraum geteilt werden kann, wobei an der Kathode die Debromierung erfolgt. Die Elektrolyse wird bei Raumtemperatur oder leicht erhöhter Temperatur durchgeführt.

Es hat sich in vielen Fällen bewährt, als Reaktionsmedium auf der Anodenseite eine wäßrige Lösung einer Säure oder eines Alkalihalogenids mit ausreichender Leitfähigkeit zu verwenden. Das Anodenmaterial wird so gewählt, daß es unter den Reaktionsbedingungen inert ist. Wird als Anolyt eine wäßrige Säure, wie z.B. Schwefelsäure verwendet, so wird vorzugsweise eine dimensionsstabile Anode oder eine Platinanode eingesetzt. Wird eine wäßrige Alkalibromidlösung Alkalibromidlösung als Anolyt verwendet, so hat sich die Verwendung einer Anode auf Kohlenstoffbasis, wie z.B. Graphit bewährt.

Auf der Kathodenseite finden niedere Alkohole als Solvens Verwendung. Vorzugsweise wird Methanol eingesetzt. Als Kathodenmaterial dient ein elektrisch leitender Werkstoff, der gegenüber halogenidhaltigen Lösungen eine ausreichende Stabilität aufweist und sich durch eine für die Debromierung der Verbindungen I ausreichend hohe Wasserstoffüberspannung auszeichnet. Vorzugsweise werden Materialien auf Kohlenstoffbasis eingesetzt, wie z.B. Graphit.

Zur Erhöhung der Wasserstoffüberspannung können dem Katholyt katalytische Mengen eines Salzes der folgenden Elemente zugesetzt werden: Cu, Ag, Au, Zn, Cd, Hg, Sn, Pb, Ti, Zr, Bi, V, Ta, Cr, Ce, Co, Ni. Wenn Salze zur Erhöhung der Wasserstoffüberspannung eingesetzt werden, dann bevorzugt Salze der Elemente Pb, Sn, Bi, Zn, Cd, Cr. Insbesondere finden Pb-Salze als Katholyt Zusatz Verwendung.
Die bevorzugten Anionen der genannten Salze sind Chlorid, Sulfat, Nitrat oder Acetat.
Die Salze können dem Elektrolyt direkt zugesetzt werden, oder auch z.B. durch Zugabe von Oxiden oder Carbonaten in der Lösung hergestellt werden. Die Salzkonzentration des Elektrolyten wird zweckmäßig auf etwa 10⁻⁶ bis 10 Gew.-%, vorzugsweise auf etwa 10⁻⁵ bis 10⁻¹, insbesondere 10⁻⁴ bis 4 x 10⁻² Gew.-%, jeweils bezogen auf die Gesamtmenge der Elektrolyselösung, eingestellt.

Die Leitfähigkeit des Elektrolyten im Kathodenraum wird durch ein Leitsalz vermittelt, das unter den Reaktionsbedingungen inert ist und das so dosiert wird, daß eine ausreichende Leitfähigkeit erzielt wird.
Beispielsweise kann ein im Elektrolyten lösliches Salz einer Carbonsäure wie etwa Natriumacetat hierfür eingesetzt werden.

Der Vorteil der Erfindung gegenüber anderen Verfahren zur Debromierung besteht darin, daß hochselektiv das Bromatom aus dem Molekül entfernt wird, ohne andere funktionelle Gruppen zu beeinträchtigen.

Die Reaktionsverfolgung kann durch gängige analytische Methoden wie z.B. Gaschromatographie erfolgen. Der Umsatz kann auch aus der übertragenen Ladungsmenge und der kathodisch gebildeten Menge Wasserstoff errechnet werden, da andere Reaktionen außer der Debromierung der Verbindungen I und Wasserstoffentwicklung nicht beobachtet werden. Insbesondere werden die Chloratome in Verbindungen des Typs I bei Überschreitung der zur vollständigen Debromierung erforderlichen Ladungsmenge nicht reduktiv eliminiert.

Die Synthese der Ausgangsverbindungen kann durch literaturbekannte Methoden ausgehend von Alkylanilinen einfach durchgeführt werden. Beispielsweise kann das in den nachfolgenden Beispielen eingesetzte 4-Brom-2,6-dichlor-3-methylacetanilid erhalten werden, indem man m-Toluidin mit Essigsäureanhydrid umsetzt. Das so erhaltene 3-Methylacetanilid wird dann mit Brom zur Reaktion gebracht, was zu einer selektiven Bromierung in der 4-Stellung führt. Die nachfolgende Umsetzung mit einem Überschuß an Chlor führt in guter Ausbeute und Selektivität zum 4-Brom-2,6-dichlor-3-methylacetanilid.

### Beispiel 1:

- Edukt:: 23,1 g 4-Brom-2,6-dichlor-3-methylacetanilid
- Katholyt:: 300 ml Methanol, 6,83 g Natriumacetat, 30-35°C
- Anolyt:: 300 ml 10 %-ige Schwefelsäure, 30-35°C
- Zelle:: geteilte Durchflußzelle, Platin-Anode (14 cm²), Graphit-Kathode (14 cm²), Nafion 417 Membran
- Stromstärke:: 1400 mA
- Ladungsmenge:: 4,17 Ah
- Umsatz:: 71 %

### Beispiel 2:

- Edukt:: 23,1 g 4-Brom-2,6-dichlor-3-methylacetanilid
- Katholyt:: 300 ml Methanol, 6,83 g Natriumacetat,
200 mg Blei(II)acetat-trihydrat, 30-35°C
- Anolyt:: 300 ml 10 %-ige Schwefelsäure, 30-35°C
- Zelle:: geteilte Durchflußzelle, Platin-Anode (14 cm²), Graphit-Kathode (14 cm²), Nafion 417 Membran
- Stromstärke:: 1400 mA
- Ladungsmenge:: 4,17 Ah
- Umsatz:: 79 %

### Beispiel 3:

- Edukt:: 23,1 g 4-Brom-2,6-dichlor-3-methylacetanilid
- Katholyt:: 300 ml Methanol, 6,83 g Natriumacetat, 30-35°C
- Anolyt:: 300 ml 30 %-ige Natiumbromidlösung, 30-35°C
- Zelle:: geteilte Durchflußzelle, Graphit-Anode (14 cm²), Graphit-Kathode (14 cm²), Nafion 417 Membran
- Stromstärke:: 1400 mA
- Ladungsmenge:: 5,63 Ah
- Umsatz:: quantitativ
- Aufarbeitung:: Der Katholyt wird mit verdünnter Schwefelsäure auf pH 4 eingestellt. Die Losung wird am Rotationsverdampfer auf etwa die Hälfte ihres ursprünglichen Volumens eingeengt und das Reaktionsprodukt durch Filtration isoliert. Man erhält 14,3 g 2,6-Dichlor-3-methylacetanilid.

## Patentansprüche

1. Verfahren zur Herstellung von 3-Alkyl-2,6-dichloracylaniliden der allgemeinen Formel I 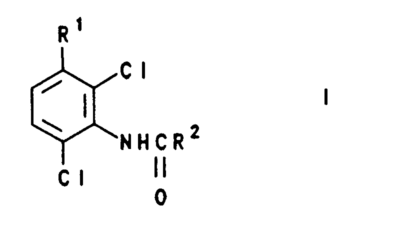 worin R¹, R² unabhängig voneinander (C₁-C₄)-Alkyl bedeuten, dadurch gekennzeichnet, daß man Bromalkylacylanilide der allgemeinen Formel II 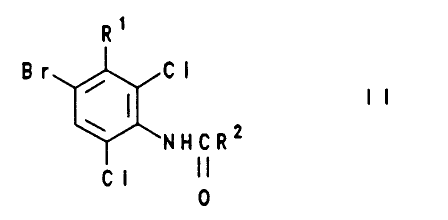 worin R¹, R² die oben angegebene Bedeutung besitzen, elektrolytisch debromiert.

2. Verfahren nach Anspruch 1, dadurch gekennzeichnet, daß R¹ und R² (C₁-C₂)-Alkyl, insbesondere Methyl, bedeuten.

3. Verfahren nach Anspruch 1 oder 2, dadurch gekennzeichnet, daß die Elektrolyse in einer Apparatur durchgeführt wird, die durch eine Membran oder ein Diaphragma abgetrennte Anoden- und Kathodenhalbräume besitzt.

4. Verfahren nach mindestens einem der Ansprüche 1 bis 3, dadurch gekennzeichnet, daß die Debromierung an der Kathode erfolgt.

5. Verfahren nach mindestens einem der Ansprüche 1 bis 4, dadurch gekennzeichnet, daß das Reaktionsmedium auf der Anodenseite eine wäßrige Lösung einer Säure oder eines Alkalihalogenids ist.

6. Verfahren nach mindestens einem der Ansprüche 1 bis 5, dadurch gekennzeichnet, daß als Anoden ein unter Reaktionsbedingungen inertes Material, eingesetzt wird.

7. Verfahren nach mindestens einem der Ansprüche 1 bis 6, dadurch gekennzeichnet, daß das Reaktionsmedium auf der Kathodenseite (Katholyt) ein (C₁-C₄)-Alkanol, insbesondere Methanol ist.

8. Verfahren nach mindestens einem der Ansprüche 1 bis 7, dadurch gekennzeichnet, daß als Kathode ein elektrisch leitendes Material, das gegenüber halogenidhaltigen Lösungen stabil ist und eine ausreichend hohe Wasserstoffüberspannung besitzt, eingesetzt wird.

9. Verfahren nach Anspruch 8, dadurch gekennzeichnet, daß als Kathode ein Material auf Kohlenstoffbasis, insbesondere Graphit eingesetzt wird.

10. Verfahren nach mindestens einem der Ansprüche 1 bis 9, dadurch gekennzeichnet, daß dem Katholyt zur Erhöhung der Wasserstoffüberspannung katalytische Mengen eines Salzes zugesetzt werden.

11. Verfahren nach Anspruch 10, dadurch gekennzeichnet, daß Salze der Metalle Cu, Ag, Au, Zn, Cd, Hg, Sn, Pb, Ti, Zr, Bi, V, Ta, Cr, Ce, Co, Ni insbesondere Pb, Sn, Bi, Zn, Cd, Cr, bevorzugt Pb eingesetzt werden.

12. Verfahren nach Anspruch 11, dadurch gekennzeichnet, daß als Salze Chloride, Sulfate, Nitrate oder Acetate eingesetzt werden.

13. Verfahren nach mindestens einem der Ansprüche 1 bis 11, dadurch gekennzeichnet, daß die Salzkonzentration des Elektrolyten auf 10⁻⁶ bis 10 Gew.-%, insbesondere 10⁻⁵ bis 10⁻¹ Gew.-%, bevorzugt 10⁻⁴ bis 4,10⁻² Gew.-%, bezogen auf die Gesamtmenge der Elektrolyselösung eingestellt wird.

14. Verfahren nach mindestens einem der Ansprüche 1 bis 13, dadurch gekennzeichnet, daß die Leitfähigkeit des Elektrolyten im Kathodenraum durch ein unter den Reaktionsbedingungen inertes Leitsalz vermittelt wird.

15. Verfahren nach Anspruch 14, dadurch gekennzeichnet, daß als Leitsalz das Salz einer Carbonsäure, insbesondere Natriumacetat eingesetzt wird.
